**Europäisches Patentamt**

**European Patent Office**

**Office européen des brevets**

(19)

(11) Numéro de publication: **0 093 663**
**B1**

# FASCICULE DE BREVET EUROPEEN

(45) Date de publication du fascicule du brevet:
**10.12.86**

(21) Numéro de dépôt: **83400851.8**

(22) Date de dépôt: **28.04.83**

(51) Int. Cl.⁴: **A 23 C 19/032** // C12N9/52

(54) Procédé destiné à améliorer l'aptitude fromagère du lait entrant dans la préparation des fromages à pâtes molles et fromages obtenus par ce procédé.

(30) Priorité: **30.04.82 FR 8207483**

(43) Date de publication de la demande: .
**09.11.83 Bulletin 83/45**

(45) Mention de la délivrance du brevet:
**10.12.86 Bulletin 86/50**

(84) Etats contractants désignés:
**AT BE CH DE FR GB IT LI NL SE**

(56) Documents cités:
**FR - A - 2 368 224**
**US - A - 3 156 568**

**CHEMICAL ABSTRACTS, vol. 70, 1969, page 23, no. 111743j, Columbus, Ohio, USA,M. DESMAZEAUD et al.: "Isolation, purification, and properties of an extracellular protease from micrococcus caseolyticus" JOURNAL OF DAIRY SCIENCE, vol. 56, no. 1, 1973, pages 33-38,V. MORENO et al.: "Degradation of beta-casein by micrococcal cell-free preparations"**

(73) Titulaire: **ROUSSEL-UCLAF, 35, boulevard des Invalides, F-75007 Paris (FR)**

(72) Inventeur: **Barthelemy, Pierre, Rue du Chamicy Rully, F-60810 Barbery (FR)**
Inventeur: **Labblee, Jean, Enil, F-25670 Mamirolle (FR)**

(74) Mandataire: **Vieillefosse, Jean-Claude et al, Département des Brevets ROUSSEL UCLAF B.P no 9, F-93230 Romainville (FR)**

ACTORUM AG

## Description

La présente invention a pour objet un procédé destiné à améliorer l'aptitude fromagère du lait entrant dans la préparation des fromages à pâtes molles, en vue de leur affinage, ainsi que les fromages obtenus par ce procédé.

L'état de la technique mentionne l'addition de préparations enzymatiques dans le préparation de certains fromages (FR-A 2 368 224); ces préparations enzymatiques présentent l'inconvénient de modifier le goût des fromages ainsi préparés.

Le document CA 70 111 793 J décrit par ailleurs la préparation d'une protéase extraite de Micrococcus caseolyticus.

On sait maintenant que les progrès techniques réalisés dans la récolte du lait, sa conservation à la ferme, son transport en citernes et son stockage à la laiterie en tanks ont de graves conséquences sur la flore bactérienne dont l'activité est essentielle pour la production d'un fromage de qualité.

La conservation du lait à 4°C a notamment pour effet de sélectionner les bactéries psychrotrophes qui produisent des acides aminés nuisant à la qualité du fromage. Cette sélection se fait au détriment de bactéries lactiques qui libèrent au cours de l'affinage les enzymes nécessaires à l'obtention de fromages de bonne qualité.

La demanderesse a ainsi été amenée à rechercher différentes voies permettant de retrouver l'aptitude fromagère comparable à celle qui existait dans les laits d'autrefois.

Au cours de ces études, elle a notamment trouvé que l'addition d'un extrait enzymatique d'une culture d'un Micrococcus caseolyticus améliorait considérablement l'aptitude fromagère du lait entrant dans la préparation des fromages à pâtes molles, en vue de leur affinage.

L'invention a ainsi pour objet un procédé destiné à améliorer l'aptitude fromagère du lait entrant dans la préparation des fromages à pâtes molles, fromages à croûte fleurie dont l'extrait sec est compris entre 30 et 50% en vue de leur affinage, procédé caractérisé en ce qu'au cours de la préparation desdits fromages à pâtes molles, l'on ajoute au lait un extrait enzymatique à activité protéolytique obtenu par centrifugation, précipation du surnageant par le sulfate d'ammonium, reprise par une solution de chlorure de calcium, concentration par ultrafiltration et lyophilisation d'un bouillon de culture de Micrococcus caséolyticus I 194, à une dose comprise entre 50 et 190 U/l de lait, l'activité protélytique de l'extrait étant déterminée comme la quantité d'extrait qui agissant dans les conditions du dosage, provoque une variation de la densité optique à 275 nm de 0,001 unité de densité optique par minute par millilitre de solution.

L'addition au lait d'un extrait enzymatique d'un Micrococcus caseolyticus répond particulièrement bien au problème posé puisqu'elle a pour effet d'une part de préparer l'action de la présure en améliorant l'aptitude à la coagulation du lait et d'autre part de créer par coupure de la caséine, des peptides stimulant des bactéries lactiques.

Grâce à cette addition d'extrait enzymatique, le fromager tend à retrouver un lait doué d'aptitude fromagère traditionnelle en reconstituant son processus de maturation biologique.

Par fromage à croûte fleurie dont l'extrait sec est compris entre 30 et 50%, on désigne notamment des fromages tels que le carré de l'est, le camembert, le brie.

Dans la mise au point du procédé, object de l'invention, on a trouvé que la souche de Micrococcus caseolyticus déposée dans la Collection Nationale de Cultures de Microorganismes (C.N.C.M.) à l'Institut Pasteur à Paris sous le no I.194 en date du 28 avril 1982 était particulièrement avantageuse.

Cette souche est identique à la souche déposée dans la collection du C.N.R.Z. (Centre National de Recherches Zootechniques à JOUY en JOSAS – FRANCE) sous le no 467 qui a par ailleurs été déjà décrite dans la littérature.

Le procédé, objet de l'invention, est donc caractérisé en ce que le micrococcus caseolyticus est constitué par la souche déposée à l'Institut Pasteur à Paris sous le no I.194.

Selon l'invention, l'addition d'extraits enzymatiques dans la préparation des fromages à pâtes molles a pour effet d'accélérer et de régulariser la protéolyse en raccourcissant la période d'affinage. Cette addition, qui a principalement pour conséquence d'avancer la période possible de commercialisation, a par ailleurs pour effet de la prolonger au-delà de la durée usuelle. Ainsi par exemple pour le carré de l'est, la période normale de commercialisation qui commence vers le 25ème jour d'affinage et se termine vers le 31ème jour peut, grâce au procédé de la présente invention, commencer vers le 21ème jour d'affinage et se terminer vers le 39ème jour d'affinage.

On doit toutefois noter que la dose d'extrait enzymatique a une importance primordiale dans la mise en œuvre de l'invention; en effet, si une dose trop faible se trouve être sans action, une dose trop importante a pour effet d'entraîner une maturation très rapide du fromage qui devient ainsi extrêmement coulant et ne peut plus être commercialisé.

Dans des conditions préférentielles de mise en œuvre du procédé, objet de l'invention, l'addition de l'extrait enzymatique est effectuée à une dose comprise entre 70 et 150 U/l de lait.

L'enzyme améliorant l'aptitude à la coagulation du lait, la dose optimale est celle qui diminue le temps de prise de 1 à 5 minutes. Cette dose, variable en fonction des qualités de laits et des fromages à préparer se situe le plus souvent aux environs de 100 U/litre.

L'activité protéolytique de l'extrait est déterminée par mesure (variation de la densité optique à 275 nm) de la quantité de caséine hydrolysée par l'enzyme (non précipitable par l'acide trichloracétique) dans des conditions standards.

Une unité est définie comme la quantité d'extrait qui agissant dans les conditions du dosage, provoque une variation de la densité optique à 275 nm de 0,001 unité de densité optique par minute par millilitre de solution.

Selon le procédé, objet de l'invention, l'addition de l'extrait est effectuée avant ou pendant l'emprésurage.

Cette addition d'extrait enzymatique est de préférence effectuée de 15 à 20 minutes avant l'emprésurage dans les cas où la température du lait est égale ou supérieure à 35°C.

Dans les cas où la température d'emprésurage n'est que de l'ordre de 30 à 32°C, température moins favorable à l'activité de l'extrait, celle-ci doit être introduite au moins 40 minutes avant l'emprésurage.

Dans les cas où la technique de fabrication des fromages ne permet pas de préserver ce temps de contact préalable, l'extrait peut être introduit dans le tank de prématuration du lait, dès la veille du jour de fabrication (lait à une température généralement comprise entre 10 et 16°C, ou dans le tank de stockage (lait à 4°C) quand la technique de prématuration n'est pas utilisée.

Une autre technique peut être appliquée dans les cas où les laits des tanks subissent une pasteurisation (72°C) ou une thermisation (63°C) juste avant la mise en route de la fabrication des fromages. Pour éviter que l'enzyme ne soit partiellement ou totalement détruite par la chaleur, la technique consiste à diluer la quantité totale d'enzyme à utiliser pour le traitement par exemple d'une cuve de 10 000 litres soit 700 000 à 1 500 000 U, dans 1 litre de lait stérile, la veille de la fabrication du fromage, cette prédilution ou préparation enzymatique étant laissée à la température ambiante soit environ 20°C.

Le préparation enzymatique est ensuite versée dans la cuve de fabrication au début du remplissage.

Cette technique a pour conséquence l'hydrolyse totale de la caséine contenue dans le litre de lait, soit environ 26 g, donnant ainsi une quantité équivalente de peptides stimulantes supplémentaires venant renforcer l'action normale de l'enzyme qui sera retée intacte dans la préparation.

Selon une variante du procédé, objet de l'invention, l'addition de l'extrait est effectuée au cours du brassage.

La technique de préparation des fromages à pâtes molles étant bien connue en elle-même, les exemples figurant plus loin dans la partie expérimentale ne sont donnés que pour illustrer l'addition d'extrait enzymatique de Micrococcus caseolyticus.

L'invention a en outre pour objet les fromages à pâtes molles tels qu'obtenus au moyen du procédé décrit ci-dessus.

Il convient de noter que le procédé selon l'invention peut être mis en œuvre aussi bien au départ du lait naturel entier, qu'au départ des laits reconstitués, recombinés ou ultrafiltrés.

Les procédés par ultrafiltration ne sont pas sans inconvénients pour l'obtention d'un fromage de qualité. En effet, en retenant la totalité des protéines et des sels minéraux dont une partie était auparavant perdue avec le sérum, l'ultrafiltration contribue à l'obtention d'un fromage plus difficile à affiner et au changement de ses caractéristiques organoleptiques.

L'addition d'extrait enzymatique de Micrococcus caseolyticus permet de pallier à ces inconvénients majeurs.

Ainsi par exemple, pour un fromage à pâte molle à croûte fleurie de type St Marcelin, la durée d'affinage qui était normalement de 24 jours a pu être ramenée à 17 jours avec l'obtention d'un produit plus mœlleux.

L'invention a enfin pour objet l'application d'un extrait enzymatique d'une culture de Micrococcus caseolyticus telle que définie ci-dessus, à la préparation de fromages à pâtes molles en vue de leur affinage.

L'extrait enzymatique de Micrococcus caseolyticus utilisée dans la mise en œuvre du procédé selon l'invention, peut par exemple être préparee selon la technique décrite dans Ann. Biol. Anim. Bioch. Biophys, 1970, 10 (3), 413–430.

L'extrait enzymatique de Micrococcus caseolyticus peut être préparée au départ d'un bouillon de culture de micrococcus caseolyticus par centrifugation dudit bouillon pour en éliminer les bactéries, précipitation de l'enzyme par le sulfate d'ammonium, reprise de celle-ci par une solution de chlorure de calcium et concentration par ultrafiltration suivie d'une lyophilisation.

Un exemple d'une telle préparation est donne ci-après:

Préparation du bouillon de fermentation

Dans un fermenteur, on stérilise à 120°C une solution préparée à partir de 750 litres d'eau, 20 kg de corn steep liquide, 20 kg de peptone de caséine, 10 kg d'autolysat de levure et 0,906 kg de chlorure de calcium dont le pH a été ajuste a 7 par addition de lessive de soude. On amène la température du milieu stérile à 30°C puis introduit 15 litres d'une solution stérile de dextrose a 30% et 10 litres de bouillon pied de cuve d'une culture de Micrococcus caseolyticus (I.194). On laisse fermenter pendant 24 heures à 30°C, sous agitation et air stérile en maintenant le pH constant pendant les 10 premières heures. Après 24 heures on recueille 1050 litres de bouillon brut de fermentation.

Extraction de l'enzyme

On sépare par centrifugation sur centrifugeuse Alfa Laval type LX les bactéries contenues dans 5000 litres d'un bouillon de brut de fermentation.

Au centrifugeat obtenu, on ajoute 2670 kg de sulfate d'ammonium, puis après agitation pendant 30 minutes, 1,2 kg d'hyflosupercel. On laisse reposer 24 heures, élimine le surnageant. On introduit la fraction insoluble obtenue ci-dessus dans 330 litres d'une solution de chlorure de calcium, agite 15 minutes et filtre. On receuille 390 litres d'une solution que l'on concentre par ultrafiltration. Après 12 heures, on obtient 50 litres d'une solution concentrée d'enzymes titrant 50 500 Unités/cm³. La solution concentrée d'enzymes est alors répartie en flacons de 24 cm³ et lyophylisée.

Il va être donné maintenant à titre non limitatif, des exemples de mise en œuvre de l'invention.

Exemple 1:

Fabrication de Carrés de l'Est au lait pasteurisé.

On traite 2000 litres de lait entier, renfermant 36 g/l de matières grasses (réfrigéré à +4°C) par des ferments lactiques et moisissures (a/ $C_5$ de Roger Penicillium Candidum lyophilisés, à raison de 5 doses pour 2000 litres, chaque dose renfermant $2.10^9$ spores, b/ 1% de mesophiles Carlin Marshall sur milieu Marstar). On ajoute du chlorure de calcium, devise en 20 bassines de 100 litres, effectue une maturation à +35°C pendant 2 heures. On ajoute alors aux 100 litres contenus dans chaque bassine 9000 unités par bassine d'enzymes de Micrococcus caseolyticus (no I.194) quinze minutes avant l'emprésurage. On ajoute alors dans chaque bassine 18 ml de présure. La prise se produit en 15 minutes (durcissement après 30 minutes et découpage du caillé en morceaux 2×2×3 cm). On procède alors à 3 brassages successifs du contenu de toutes les bassines avec un temps de repos de 6 minutes entre chaque brassage. On élimine une partie de sérum exsudé après caillage (acidité du sérum 42° Dornic) puis procède au moulage et à l'égouttage. On effectue un premier retournement après l'égouttage, un second retournement 15 minutes après, un troisième retournement 3 heures après le second et enfin un quatrième retournement 4 heures après le troisième (acidité du sérum 70° D). On procède à un démoulage 24 heures après le début de l'opération et obtient 960 carrés de l'est (pH du fromage 4,70 – acidité du sérum 115° D). Les fromages obtenus étaient commercialisables et consommables après 21 jours d'affinage; ils l'étaient encore au détail après 39 jours d'affinage. Ces résultats montrent que la période de commercialisation au détail qui est normalement de 12 jours peut être allongée à 24 jours en utilisant le procédé selon l'invention.

Exemple 2:

Fabrication de Carrés de l'Est au lait pasteurisé.

On traite 2000 litres de lait entier, renfermant 28 g/l de matières grasses réfrigéré à +4°C, par des ferments lactiques et moisissures (spores de penicillium candidum lyophilisés 5 doses pour 2000 litres: $2.10^9$ spores, 1% mesophiles Carlin Marshall sur milieu Marstar), du chlorure de calcium, divise en 20 bassines de 100 litres et effectue une maturation à 35°C pendant 2 heures. On ajoute alors aux 100 litres contenus dans chacune des 4 bassines dites traitées, respectivement des doses de 9000, 10 000, 11 000 et 12 000 Unités d'enzymes de Micrococcus caseolyticus (no I.194) quinze minutes avant l'emprésurage. On ajoute alors au contenu des 20 bassines (4 traitées et 16 non traitées servant de témoins) 18 ml de présure. La prise a lieu en 20 minutes pour le contenu des bassines non traitées, en 17 minutes pour la bassine traitée par 9000 U d'enzymes et en 16 minutes pour les bassines traitées

par 10 000, 11 000 et 12 000 unités d'enzymes. On procède alors à 3 brassages successifs du contenu de toutes les bassines avec un temps de repos de 6 minutes entre chaque brassage. On élimine une partie du sérum exsudé après caillage. On procède alors au moulage puis à l'égouttage. On effectue un premier retournement des fromages après le moulage puis un second 15 minutes après le premier. On effectue un troisième retournement 3 heures après la fin du second et enfin un quatrième retournement 4 heures après le troisième. L'acidité du sérum est de 65° Dornic (°D) pour les fromages non traités servant de témoins, de 64°D pour les fromages traités par 9000 U d'enzymes, de 65°D pour les fromages traités par 10 000 U d'enzymes, de 70°D pour les fromages traités par 11 000 U d'enzymes et de 70°D pour les fromages traités par 12 000 U d'enzymes. On procède alors à un démoulage 24 heures après le début du traitement et obtient 960 carrés de l'est. Le pH des fromages témoins et des fromages traités au four J + l figure dans le tableau ci-après.

| Fromages | | | | |
|---|---|---|---|---|
| témoins | traités par × 1000 U d'enzymes | | | |
| | 9 | 10 | 11 | 12 |
| 4,80 | 4,90 | 4,90 | 4,90 | 4,9-0 |

Ce n'est que vers le 25ème jour d'affinage que les fromages témoins ont eu une texture analogue à celle des fromages traités, au 21ème jour d'affinage.

Les fromages traités étaient encore parfaitement consommables après 39 jours d'affinage alors que dès le 31ème jour les fromages témoins étaient protéolysés en excès et avaient une saveur d'ammoniaque.

Exemple 3:

Fabrication de Carrés de l'Est.

On traite 2100 litres de lait entier, renfermant 36 g/l de matières grasses, réfrigéré à +4°C, par des ferments lactiques et moisissures (spores de penicillium candidum lyophilisées 5 doses, 1% mesophiles Carlin Marshall sur milieu Marstar), du chlorure de calcium, divise en 21 bassines de 100 litres, effectue une maturation à 35°C pendant 2 heures. On ajoute alors aux 100 litres contenus dans chacune des 9 bassines (dites traitées), respectivement des doses de 7000, 8000, 9000, 10 000, 11 000, 12 000, 13 000, 14 00, 15 000 unités par bassine d'enzyme de Micrococcus caseolyticus (no I.194) quinze minutes avant l'emprésurage. On ajoute alors au contenu des 21 bassines (9 traitées et 12 non traitées) 20 ml de présure. La prise se produit en 15 minutes pour les bassines non traitées et en 12 à 15 minutes pour les bassines traitées (plus rapidement pour la bassine traitée par 15 000 unités d'enzymes

pour 100 litres). On procède alors à 3 brassages successifs du contenu de toutes les bassines avec un temps de repos de 6 minutes entre chaque brassage. On élimine une partie du sérum exsudé après caillage. On procède alors au moulage puis à l'égouttage. On effectue un premier retournement après le moulage, puis un second 15 minutes après la fin du moulage. On effectue un troisième retournement 3 heures après la fin du second et enfin un quatrième retournement 4 heures après la fin du troisième. Après le 4ème retournement, l'acidité du sérum est de 65°D pour les fromages témoins, de 55°D pour les fromages traités par 7000, 8000 et 9000 U d'enzymes et de 58°D pour les fromages traités par 10, 11, 12, 13, 14 et 15 000 U d'enzymes. On procède à un démoulage 24 heures après le début du traitement et obtient 1008 carrés de l'est. Le pH des fromages témoins et des fromages traités aux jours J + l et J + 4 figure dans le tableau ci-après.

Fromages

| temps | témoins | traités par × 1000 U/d'enzymes | | | | | | | | |
|---|---|---|---|---|---|---|---|---|---|---|
| | | 7 | 8 | 9 | 10 | 11 | 12 | 13 | 14 | 15 |
| J + 1 | 4,87 | 4,88 | 4,85 | 4,87 | 4,86 | 4,81 | 4,83 | 4,82 | 4,80 | 4,75 |
| J + 4 | 4,60 | 4,59 | 4,63 | 4,64 | 4,68 | 4,65 | 4,63 | 4,67 | 4,68 | 4,67 |

Après un affinage de 21 jours les fromages témoins et traités ont été dégustés. Dans chaque cas, on a noté l'aspect et le goût. Les résultats de cette dégustation figurent dans le tableau ci-après.

| fromages | aspect et goût après 21 jours d'affinage |
|---|---|
| témoins | pâte blanche et ferme peu protéolysée |
| traités 7000 U | onctueux léger goût de protéolyse |
| traités 8000 U | onctueux coulant légerement sous croûte |
| traités 9000 U | pâte assez ferme et homogène goût lactique |
| traités 10 000 U | pâte homogène goût plat |
| traités 11 000 U | coulant légèrement sous croûte léger goût de protéolyse |
| traités 12 000 U | onctueux – coulant sous croûte goût acide |
| traités 13 000 U | très coulant |
| traités 14 000 U | coulant sous croûte goût acide |
| traités 15 000 U | très coulant |

Les résultats obtenus montrent que l'affinage des fromages traités est plus précoce que l'affinage des fromages témoins. Dans l'ensemble, la protéolyse est très rapide pour les fromages traités par une forte teneur en enzymes.

**Revendications pour les Etats contractants BE, CH, DE, FR, GB, IT, LI, NL, SE**

1. Procédé destiné à améliorer l'aptitude fromagère du lait entrant dans la préparation des fromages à pâtes molles, fromages à croûte fleurie dont l'extrait sec est compris entre 30 et 50%, en vue de leur affinage, procédé caractérisé en ce qu'au cours de la préparation desdits fromages à pâtes molles, l'on ajoute au lait un extrait enzymatique à activité protéolytique obtenu par centrifugation, précipitation du surnageant par le sulfate d'ammonium, reprise par une solution de chlorure de calcium, concentration par ultrafiltration et lyophilisation d'un bouillon de culture de Micrococcus caséolyticus I 194, à une dose comprise entre 50 et 190 U/l de lait, l'activité protéolytique de l'extrait étant déterminée comme la quantité d'extrait qui agissant dans les conditions du dosage, provoque une variation de la densité optique à 275 nm de 0,001 unité de densité optique par minute par millilitre de solution.

2. Procédé selon la revendication 1, caractérisé en ce que l'addition de l'extrait enzymatique est effectuée à une dose comprise entre 70 et 150 U/l de lait.

3. Fromage a pâte molle obtenu au moyen du procédé décrit à l'une quelconque des revendications 1 ou 2.

**Revendications pour l'Etat contractant AT**

1. Procédé destiné à améliorer l'aptitude fromagère du lait entrant dans la préparation des fromages à pâtes molles, fromages à croûte fleurie dont l'extrait sec est compris entre 30 et 50%, en vue de leur affinage, procédé caractérisé en ce qu'au cours de la préparation desdits fromages à pâtes molles, l'on ajoute au lait un extrait enzymatique à activité protéolytique obtenu par centrifugation, précipitation du surnageant par le sulfate d'ammonium, reprise par une solution de chlorure de calcium, concentration par ultrafiltration et lyophilisation d'un bouillon de culture de Micrococcus caséolyticus I 194, à une dose comprise entre 50 et 190 U/l de lait, l'activité protéolytique de l'extrait étant déterminée comme la quantité d'extrait qui agissant dans les conditions du dosage, provoque une variation de la densité optique à 275 nm de 0,001 unité de densité optique par minute par millilitre de solution.

2. Procédé selon la revendication 1, caractérisé en ce que l'addition de l'extrait enzymatique est effectuée à une dose comprise entre 70 et 150 U/l de lait.

## Patentansprüche für die Vertragsstaaten: BE, CH, DE, FR, GB, IT, LI, NL, SE

1. Verfahren zur Verbesserung der Käsetauglichkeit von Milch, die in die Herstellung von Käsen aus Weichkäsemassen, Käsen mit frischer Rinde, deren Trockenextrakt zwischen 30 und 50% liegt, eingeht im Hinblick auf deren Veredelung, dadurch gekennzeichnet, dass man im Verlauf der Herstellung dieser Käse aus Weichkäsemassen zu der Milch einen Enzymextrakt mit proteolytischer Aktivität, erhalten durch Zentrifugieren, Ausfällen des Überstands mit Ammoniumsulfat, Wiederaufnahme mit einer Calciumchloridlösung, Einengen durch Ultrafiltration und Lyophilisierung einer Kulturbrühe von Micrococcus caseolyticus I 194, in einer Dosis zwischen 50 und 190 E/l Milch zugibt, wobei die proteolytische Aktivität des Extrakts als die Extraktmenge bestimmt wird, die unter den Bestimmungsbedingungen eine Änderung der optischen Dichte bei 275 nm von 0,001 Einheiten optischer Dichte je Minute je Milliliter Lösung herbeiführt.

2. Verfahren gemäss Anspruch 1, dadurch gekennzeichnet, dass die Zugabe des Enzymextrakts in einer Dosis zwischen 70 und 150 E/l Milch durchgeführt wird.

3. Käse aus Weichkäsemassen, erhalten mit Hilfe eines Verfahrens gemäss einem der Ansprüche 1 oder 2.

## Patentansprüche für den Vertragsstaat: AT

1. Verfahren zur Verbesserung der Käsetauglichkeit von Milch, die in die Herstellung von Käsen aus Weichkäsemassen, Käsen mit frischer Rinde, deren Trockenextrakt zwischen 30 und 50% liegt, eingeht, im Hinblick auf deren Veredelung, dadurch gekennzeichnet, dass man im Verlauf der Herstellung dieser Käse aus Weichkäsemassen zu der Milch einen Enzymextrakt mit proteolytischer Aktivität, erhalten durch Zentrifugieren, Ausfällen des Überstands mit Ammoniumsulfat, Wiederaufnahme mit einer Calciumchloridlösung, Einengen durch Ultrafiltration und Lyophilisieren einer Kulturbrühe von Micrococcus caseolyticus I 194, in einer Dosis zwischen 50 und 190 E/l Milch zugibt, wobei die proteolytische Aktivität des Extrakts als die Extraktmenge bestimmt wird, die unter den Bestimmungsbedingungen eine Änderung der optischen Dichte bei 275 nm von 0,001 Einheiten optischer Dichte je Minute je Milliliter Lösung herbeiführt.

2. Verfahren gemäss Anspruch 1, dadurch gekennzeichnet, dass die Zugabe des Enzymextrakts in einer Dosis zwischen 70 und 150 E/l Milch durchgeführt wird.

## Claims for the contracting states BE, CH, DE, FR, IT, LI, NL, SE, GB

1. Process intended to improve the cheese-making aptitude of the milk entering into the preparation of soft paste cheeses, cheeses with flowery crust of which the dry extract is between 30 and 50%, for the purpose of maturing them, which process is characterized in that in the course of the preparation of these said soft paste cheeses, there is added to the milk an enzyme extract with proteolytic activity obtained by centrifuging, precipitation of the supernatant by ammonium sulphate, taking up with a calcium chloride solution, concentration by ultrafiltration and lyophilisation of a culture broth of Micrococcus caseolyticus I 194, at a dosage between 50 and 190 U/l. of milk, the proteolytic activity of the extract being determined as the quantity of the extract which acting in the conditions of the dosage causes a variation of the optical density at 275 nm of 0.001 units of optical density per minute per millilitre of solution.

2. Process according to claim 1, characterized in that the addition of the enzyme extract is carried out at a dose between 70 and 150 U/l. of milk

3. Soft paste cheese obtained by means of the process described in either of the claims 1 or 2

## Claims for the contracting State AT

1. Process intended to improve the cheese-making aptitude of the milk entering into the preparation of soft paste cheeses, cheeses with flowery crust of which the dry extract is between 30 and 50%, for the purpose of maturing them, which process is characterized in that the course of the preparation of these said soft paste cheeses, there is added to the milk an enzyme extract with proteolytic activity obtained by centrifuging, precipitation of the supernatant by ammonium sulphate, taking up with a calcium chloride solution, concentration by ultrafiltration and lyophilisation of a culture broth of Micrococcus caseolyticus I 194, at a dosage between 50 and 190 U/l. of milk, the proteolytic activity of the extract being determined as the quantity of the extract which acting in the conditions of the dosage causes a variation of the optical density at 275 nm of 0.001 units of optical density per minute per millilitre of solution.

2. Process according to claim 1, characterized in that the addition of the enzyme extract is carried out at a dose between 70 and 150 U/l. of milk